# EUROPEAN PATENT SPECIFICATION

(11) **EP 2 480 250 B1**
(45) Date of publication and mention of the grant of the patent: **16.04.2014**
(21) Application number: 10761093.3
(22) Date of filing: 22.09.2010
(51) Int. Cl.: A61K 38/48, A61P 19/10

(54) **Compositions comprising botulinum toxin A or B for use in the treatment of osteoporosis**
Zusammensetzungen Botulinumtoxin A oder B enthaltend zur Verwendung bei der Behandlung der Osteoporose
Compositions comprenant des toxines botuliques A ou B pour l'utilisation dans la traitement de l'ostéoporose

(30) Priority: 24.09.2009 US 245595 P
(43) Date of publication of application: 01.08.2012
(73) Proprietor: Allergan, Inc., Irvine, CA 92612 (US)
(72) Inventor: RIMANDO, Marlon P., Livingston New Jersey 07039 (US)
(74) Representative: HOFFMANN EITLE
(86) International application number: PCT/US2010/049844
(87) International publication number: WO 2011/038015

(56) References cited:
- GB-A- 2 400 316
- CHADDOCK J A ET AL: "Clostridial neurotoxins: structure-function led design of new therapeutics", CMLS CELLULAR AND MOLECULAR LIFE SCIENCES, vol. 63, no. 5, 16 January 2006 (2006-01-16), pages 540-551, XP002376630, BIRKHAUSER VERLAG, HEIDELBERG, DE ISSN: 1420-682X, DOI: 10.1007/S00018-005-5505-5
- GRIMSTON SUSAN K ET AL: "Bone loss after temporarily induced muscle paralysis by Botox is not fully recovered after 12 weeks.", ANNALS OF THE NEW YORK ACADEMY OF SCIENCES, vol. 1116, November 2007 (2007-11), pages 444-460, XP002618083, ISSN: 0077-8923
- BLOUIN S ET AL: "Disuse and orchidectomy have additional effects on bone loss in the aged male rat.", OSTEOPOROSIS INTERNATIONAL : A JOURNAL ESTABLISHED AS RESULT OF COOPERATION BETWEEN THE EUROPEAN FOUNDATION FOR OSTEOPOROSIS AND THE NATIONAL OSTEOPOROSIS FOUNDATION OF THE USA, vol. 18, no. 1, January 2007 (2007-01), pages 85-92, XP002618081, ISSN: 0937-941X
- WARNER SARAH E ET AL: "Botox induced muscle paralysis rapidly degrades bone.", BONE, vol. 38, no. 2, February 2006 (2006-02), pages 257-264, XP002618082, ISSN: 8756-3282
- YASOTHAN UMA ET AL: "Osteoporosis: overview and pipeline.", NATURE REVIEWS. DRUG DISCOVERY, vol. 7, no. 9, September 2008 (2008-09), pages 725-726, XP002618084, ISSN: 1474-1776
- YADAV VIJAY K ET AL: "Pharmacological inhibition of gut-derived serotonin synthesis is a potential bone anabolic treatment for osteoporosis.", NATURE MEDICINE, vol. 16, no. 3, March 2010 (2010-03), pages 308-312+1P, XP002618085, ISSN: 1546-170X
- YADAV VIJAY K ET AL: "Lrp5 controls bone formation by inhibiting serotonin synthesis in the duodenum.", CELL, vol. 135, no. 5, 28 November 2008 (2008-11-28), pages 825-837, XP002618212, ISSN: 1097-4172
- Seth L Matarasso: "Comparison of Botulinum Toxin Types A And B: A Bilateral and Double-Blind Randomized Evaluation in the Treatment of Canthal Rhytides", Dermatologic Surgery, vol. 29, no. 1, 1 January 2003 (2003-01-01), pages 7-13, XP055051688, ISSN: 1076-0512, DOI: 10.1046/j.1524-4725.2003.29017.x

## Description

### CROSS REFERENCE

This application claims the benefit of U.S. Provisional Patent Application Serial Number 61/245,595, filed on September 24, 2009.

### BACKGROUND OF THE INVENTION

Osteoporosis is a bone disease that leads to an increased risk of fracture. In osteoporosis, bone mineral density (BMD) is reduced, bone microarchitecture is disrupted, and the amount and variety of non-collagenous proteins in bone is altered. Osteoporosis is most common in women but may also develop in men as well. Due to the increased associated risk of fragility fracture, osteoporosis may significantly affect life expectancy and quality of life.

The underlying mechanism in osteoporosis is an imbalance between bone resorption and bone formation. In normal bone, there is constant matrix remodeling of bone; up to 10% of all bone mass may be undergoing remodeling at any point in time. Bone is resorbed by osteoclast cells (which derive from the bone marrow), after which new bone is deposited by osteoblast cells. Numerous hormones play a role in bone deposition and resorption. Recently, a prominent role of gut-derived serotonin (GDS) in bone formation has been elucidated. Most circulating serotonin is synthesized in the duodenum by specialized neuroendocrine enterochromaffin cells in a process involving the enzyme tryptophan hydroxylase 1 (Tph1). Studies show that patients taking selective serotonin reuptake inhibitors (SSRIs) for treatment of depression, which increase extracellular serotonin concentration throughout the body, exhibited decreased bone mass and increased incidence of bone fractures. It is believed that GDS mediates its effect on bone formation by inhibiting osteoblast activity.

The link between GDS and bone formation provides a new target for treating osteoporosis. A method of treating osteoporosis by inhibiting or reducing GDS production would be desirable. Further, a neurotoxin useful for inhibiting or reducing GDS production would be highly desirable.

The genus *Clostridium* has more than one hundred and twenty seven species, grouped according to their morphology and functions. The anaerobic, gram positive bacterium *Clostridium botulinum* produces a potent polypeptide neurotoxin, botulinum toxin, which causes a neuroparalytic illness in humans and animals referred to as botulism. The spores of *Clostridium botulinum* are found in soil and can grow in improperly sterilized and sealed food containers of home based canneries, which are the cause of many of the cases of botulism. The effects of botulism typically appear 18 to 36 hours after eating the foodstuffs infected with a *Clostridium botulinum* culture or spores. The botulinum toxin can apparently pass unattenuated through the lining of the gut and shows a high affinity for cholinergic motor neurons. Symptoms of botulinum toxin intoxication can progress from difficulty walking, swallowing, and speaking to paralysis of the respiratory muscles and death.

About 50 picograms of a commercially available botulinum toxin type A (purified neurotoxin complex) has an LD₅₀ in mice (i.e. 1 unit). One unit of BOTOX® (botulinum toxin type A, Allergan, Inc., Irvine, CA) contains about 50 picograms (about 56 attomoles) of botulinum toxin type A complex. Interestingly, on a molar basis, botulinum toxin type A is about 1.8 billion times more lethal than diphtheria, about 600 million times more lethal than sodium cyanide, about 30 million times more lethal than cobra toxin and about 12 million times more lethal than cholera (Singh, Critical Aspects of Bacterial Protein Toxins, pages 63-84 (chapter 4) of Natural Toxins II, edited by B R. Singh et al., Plenum Press, New York (1976) (where the stated LD₅₀ of botulinum toxin type A of 0.3 ng equals 1 U is corrected for the fact that about 0.05 ng of BOTOX® equals 1 unit)). One unit (U) of botulinum toxin is defined as the LD₅₀ upon intraperitoneal injection into female Swiss Webster mice weighing 18 to 20 grams each. (Available from Allergan, Inc., of Irvine, Calif. under the tradename BOTOX® in 100 unit vials).

Seven generally immunologically distinct botulinum neurotoxins have been characterized, these being respectively botulinum neurotoxin serotypes A, B, C₁, D, E, F and G, each of which is distinguished by neutralization with type-specific antibodies. The different serotypes of botulinum toxin vary in the animal species that they affect and in the severity and duration of the paralysis they evoke. For example, it has been determined that botulinum toxin type A is 500 times more potent, as measured by the rate of paralysis produced in the rat, than is botulinum toxin type B. Additionally, botulinum toxin type B has been determined to be non-toxic in primates at a dose of 480 U/kg which is about 12 times the primate LD₅₀ for botulinum toxin type A (Moyer E et al., Botulinum Toxin Type B: Experimental and Clinical Experience, being chapter 6, pages 71-85 of "Therapy With Botulinum Toxin", edited by Jankovic, J. et al. (1994), Marcel Dekker, Inc.).

Regardless of serotype, the molecular mechanism of toxin intoxication appears to be similar and involve at least three steps or stages. In the first step of the process, the toxin binds to the presynaptic membrane of the target neuron through a specific interaction between the heavy chain (the H chain or HC), and a cell surface receptor. In the second step, the botulinum toxin crosses the plasma membrane of the target cell. The botulinum toxin is first engulfed by the cell through receptor-mediated endocytosis, and an endosome containing the botulinum toxin is formed. The toxin then escapes the endosome into the cytoplasm of the cell. The last step of the mechanism of botulinum toxin activity appears to involve reduction of the disulfide bond joining the heavy chain, H chain, and the light chain, L chain. The entire toxic activity of botulinum and tetanus toxins is contained in the L chain of the holotoxin; the L chain is a zinc (Zn²⁺) endopeptidase which selectively cleaves proteins essential for recognition and docking of neurotransmitter-containing vesicles with the cytoplasmic surface of the plasma membrane, and fusion of the vesicles with the plasma membrane. Tetanus neurotoxin, botulinum toxin types B, D, F, and G cause degradation of synaptobrevin (also called vesicle-associated membrane protein (VAMP)), a synaptosomal membrane protein. Most of the VAMP present at the cytoplasmic surface of the synaptic vesicle is removed as a result of any one of these cleavage events. Botulinum toxin serotype A and E cleave synaptosomal associated protein (SNAP-25). Botulinum toxin serotype C₁ was originally thought to cleave syntaxin, but was found to cleave syntaxin and SNAP-25. Each of the botulinum toxins specifically cleaves a different bond, except botulinum toxin type B (and tetanus toxin) which cleave the same bond. Each of these cleavages block the process of vesicle-membrane docking, thereby preventing exocytosis of vesicle content.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum types A and E both cleave the 25 kiloDalton (kD) SNAP-25, but they target different amino acid sequences within this protein. Botulinum toxin types B, D, F and G act on vesicle-associated protein (VAMP), with each serotype cleaving the protein at a different site. Finally, botulinum toxin type C₁ has been shown to cleave both syntaxin and SNAP-25. These differences in mechanism of action may affect the relative potency and/or duration of action of the various botulinum toxin serotypes. Apparently, a substrate for a botulinum toxin can be found in a variety of different cell types.

The molecular weight of the botulinum toxin protein molecule, for all seven of the known botulinum toxin serotypes, is about 150 kD. Interestingly, the botulinum toxins are released by *Clostridial* bacterium as complexes comprising the 150 kD botulinum toxin protein molecule along with associated non-toxin proteins. Thus, the botulinum toxin type A complex can be produced by *Clostridial* bacterium as 900 kD, 500 kD and 300 kD forms. Botulinum toxin types B and C₁ are apparently produced as only a 700 kD or 500 kD complex. Botulinum toxin type D is produced as both 300 kD and 500 kD complexes. Finally, botulinum toxin types E and F are produced as only approximately 300 kD complexes. The complexes (i.e. molecular weight greater than about 150 kD) are believed to contain a non-toxin hemaglutinin proteins and a non-toxin and non-toxic nonhemaglutinin protein. These two non-toxin proteins (which along with the botulinum toxin molecule comprise the relevant neurotoxin complex) may act to provide stability against denaturation to the botulinum toxin molecule and protection against digestive acids when a botulinum toxin is ingested. Additionally, it is possible that the larger (greater than about 150 kD molecular weight) botulinum toxin complexes may result in a slower rate of diffusion of the botulinum toxin away from a site of injection of a botulinum toxin complex.

Botulinum toxin type A can be obtained by establishing and growing cultures of *Clostridium botulinum* in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the botulinum toxin type B serotype only cleave a portion of the toxin produced. The exact proportion of nicked to unnicked molecules depends on the length of incubation and the temperature of the culture. Therefore, a certain percentage of any preparation of, for example, the botulinum toxin type B toxin is likely to be inactive, possibly accounting for the known significantly lower potency of botulinum toxin type B as compared to botulinum toxin type A. The presence of inactive botulinum toxin molecules in a clinical preparation will contribute to the overall protein load of the preparation, which has been linked to increased antigenicity, without contributing to its clinical efficacy.

High quality crystalline botulinum toxin type A can be produced from the Hall A strain of *Clostridium botulinum* with characteristics of ≤3x10⁷ U/mg, an A₂₆₀/A₂₇₈ of less than 0.60 and a distinct pattern of banding on gel electrophoresis. The known Shantz process can be used to obtain crystalline botulinum toxin type A, as set forth in Shantz, E. J., et al. (Properties and use of Botulinum toxin and Other Microbial Neurotoxins in Medicine, Microbiol Rev., 56: 80-99, 1992). Generally, the botulinum toxin type A complex can be isolated and purified from an anaerobic fermentation by cultivating Clostridium botulinum type A in a suitable medium. The known process can also be used, upon separation out of the non-toxin proteins, to obtain pure botulinum toxins, such as for example: purified botulinum toxin type A with an approximately 150 kD molecular weight with a specific potency of 1-2x10⁸ LD₅₀ U/mg or greater; purified botulinum toxin type B with an approximately 156 kD molecular weight with a specific potency of 1-2x10⁸ LD₅₀ U/mg or greater, and; purified botulinum toxin type F with an approximately 155 kD molecular weight with a specific potency of 1-2x10⁷ LD₅₀ U/mg or greater.

As with enzymes generally, the biological activities of the botulinum toxins (which are intracellular peptidases) are dependant, at least in part, upon their three-dimensional conformation. Thus, botulinum toxin type A is detoxified by heat, various chemicals surface stretching and surface drying. Additionally, it is known that dilution of a botulinum toxin complex obtained by the known culturing, fermentation and purification to the much, much lower toxin concentrations used for pharmaceutical composition formulation results in rapid detoxification of the toxin unless a suitable stabilizing agent is present. Dilution of the toxin from milligram quantities to a solution containing nanograms per milliliter presents significant difficulties because of the rapid loss of specific toxicity upon such great dilution. Since the botulinum toxin may be used months or years after the toxin containing pharmaceutical composition is formulated, the toxin can be stabilized with a stabilizing agent such as albumin and/or gelatin and /or sugars.

A commercially available botulinum toxin containing pharmaceutical composition is sold under the trademark BOTOX® (available from Allergan, Inc., of Irvine, Calif.). BOTOX® consists of a purified botulinum toxin type A complex, albumin and sodium chloride packaged in sterile, vacuum-dried form. The botulinum toxin type A is made from a culture of the Hall strain of *Clostridium botulinum* grown in a medium containing N-Z amine and yeast extract. The botulinum toxin type A complex is purified from the culture solution by a series of acid precipitations to a crystalline complex consisting of the active high molecular weight toxin protein and an associated hemagglutinin protein. The crystalline complex is re-dissolved in a solution containing saline and albumin and sterile filtered (0.2 microns) prior to vacuum-drying. The vacuum-dried product is stored in a freezer at or below -5°C. BOTOX® can be reconstituted with sterile, non-preserved saline prior to intramuscular injection. Each vial of BOTOX® contains about 100 units (U) of *Clostridium botulinum* toxin type A purified neurotoxin complex, 0.5 milligrams of human serum albumin and 0.9 milligrams of sodium chloride in a sterile, vacuum-dried form without a preservative.

To reconstitute vacuum-dried BOTOX®, sterile normal saline without a preservative, (0.9% Sodium Chloride Injection) is used by drawing up the proper amount of diluent in the appropriate size syringe. Since BOTOX® may be denatured by bubbling or similar violent agitation, the diluent is gently injected into the vial. For sterility reasons BOTOX® is preferably administered within twenty four (24) hours after the vial is removed from the freezer and reconstituted. During these twenty four (24) hours, reconstituted BOTOX® can be stored in a refrigerator at about 2°C to about 8°C. Reconstituted, refrigerated BOTOX® has been reported to retain its potency for at least about two weeks.

It has been reported that botulinum toxin type A has been used in clinical settings as follows:
(1) about 75-125 units of BOTOX® per intramuscular injection (multiple muscles) to treat cervical dystonia;
(2) 5-10 units of BOTOX® per intramuscular injection to treat glabellar lines (brow furrows) (5 units injected intramuscularly into the procerus muscle and 10 units injected intramuscularly into each corrugator supercilii muscle);
(3) about 30-80 units of BOTOX® to treat constipation by intrasphincter injection of the puborectalis muscle;
(4) about 1-5 units per muscle of intramuscularly injected BOTOX® to treat blepharospasm by injecting the lateral pre-tarsal orbicularis oculi muscle of the upper lid and the lateral pre-tarsal orbicularis oculi of the lower lid;
(5) to treat strabismus, extraocular muscles have been injected intramuscularly with between about 1-5 units of BOTOX®, the amount injected varying based upon both the size of the muscle to be injected and the extent of muscle paralysis desired (i.e. amount of diopter correction desired);
(6) to treat upper limb spasticity following stroke by intramuscular injections of BOTOX® into five different upper limb flexor muscles, as follows:
   (a) flexor digitorum profundus: 7.5 U to 30 U
   (b) flexor digitorum sublimus: 7.5 U to 30 U
   (c) flexor carpi ulnaris: 10 U to 40 U
   (d) flexor carpi radialis: 15 U to 60 U
   (e) biceps brachii: 50 U to 200 U. Each of the five indicated muscles has been injected at the same treatment session, so that the patient receives from 90 U to 360 U of upper limb flexor muscle BOTOX® by intramuscular injection at each treatment session; and
(7) to treat migraine, pericranial injected (injected symmetrically into glabellar, frontalis and temporalis muscles) injection of 25 U of BOTOX® has showed significant benefit as a prophylactic treatment of migraine compared to vehicle as measured by decreased measures of migraine frequency, maximal severity, associated vomiting and acute medication use over the three month period following the 25 U injection.

It is known that botulinum toxin type A can have an efficacy for up to 12 months, and in some circumstances for as long as 27 months, when used to treat glands, such as in the treatment of hyperhydrosis. However, the usual duration of an intramuscular injection of BOTOX® is typically about 3 to 4 months.

The success of botulinum toxin type A to treat a variety of clinical conditions has led to interest in other botulinum toxin serotypes. Two commercially available botulinum type A preparations for use in humans are BOTOX® available from Allergan, Inc., of Irvine, Calif., and DYSPORT® available from Beaufour Ipsen, Porton Down, England. A Botulinum toxin type B preparation (MYOBLOC®) is available from Solstice Neuroscience, South San Francisco, CA, USA.

A botulinum toxin has also been proposed for or has been used to treat skin wounds (U.S. Pat. No. 6,447,787), various autonomic nerve dysfunctions (U.S. Pat. No. 5,766,605), tension headache, (U.S. Pat. No. 6,458,365), migraine headache pain (U.S. Pat. No. 5,714,468), post-operative pain and visceral pain (U.S. Pat. No. 6,464,986), hair growth and hair retention (U.S. Pat. No. 6,299,893), psoriasis and dermatitis (U.S. Pat. No. 5,670,484), injured muscles (U.S. Pat. No. 6,423,319) various cancers (U.S. Pat. No. 6,139,845), smooth muscle disorders (U.S. Pat. No. 5,437,291), nerve entrapment syndromes (U.S. Published Patent Application 20030224019, filed February 27, 2003), acne (WO 03/011333) and neurogenic inflammation (U.S. Pat. No. 6,063,768). Controlled release toxin implants are known (see e.g. U.S. Pat. Nos. 6,306,423 and 6,312,708) as is transdermal botulinum toxin administration (U.S. Published Patent Application No. 20040009180, filed July 11, 2002).

It is known that a botulinum toxin can be used to weaken the chewing or biting muscle of the mouth so that self inflicted wounds and resulting ulcers can heal; permit healing of benign cystic lesions or tumors; treat anal fissure; and treat certain types of atopic dermatitis.

Additionally, a botulinum toxin may have an effect to reduce induced inflammatory pain in a rat formalin model. Furthermore, it has been reported that botulinum toxin nerve blockage can cause a reduction of epidermal thickness. Finally, it is known to administer a botulinum toxin to the foot to treat excessive foot sweating, spastic toes, idiopathic toe walking, and foot dystonia.

Tetanus toxin, as wells as derivatives (i.e. with a non-native targeting moiety), fragments, hybrids and chimeras thereof can also have therapeutic utility. The tetanus toxin bears many similarities to the botulinum toxins. Thus, both the tetanus toxin and the botulinum toxins are polypeptides made by closely related species of Clostridium (*Clostridium tetani* and *Clostridium botulinum,* respectively). Additionally, both the tetanus toxin and the botulinum toxins are dichain proteins composed of a light chain (molecular weight about 50 kD) covalently bound by a single disulfide bond to a heavy chain (molecular weight about 100 kD). Hence, the molecular weight of tetanus toxin and of each of the seven botulinum toxins (non-complexed) is about 150 kD. Furthermore, for both the tetanus toxin and the botulinum toxins, the light chain bears the domain which exhibits intracellular biological (protease) activity, while the heavy chain comprises the receptor binding (immunogenic) and cell membrane translocational domains.

Tetanus toxin and the botulinum toxins resemble each other in both biosynthesis and molecular architecture. Thus, there is an overall 34% identity between the protein sequences of tetanus toxin and botulinum toxin type A, and a sequence identity as high as 62% for some functional domains.

As stated above, the link between GDS and bone formation provides a new opportunity and means for treating osteoporosis. Treating osteoporosis by inhibiting or reducing GDS production, particularly with a neurotoxin (such as a botulinum toxin, for example), would be highly desirable.

### SUMMARY

An embodiment of the present invention is a composition comprising a neurotoxin for use in a method of treating osteoporosis in a mammal, wherein the neurotoxin is selected from botulinum toxin types A and B.

As used herein, the following definitions are provided.

About: as used herein "about" means approximately or nearly and in the context of a numerical value or range set forth means ±15% of the numerical value range recited or claimed.

Enhancing agent: as used herein "enhancing agent" refers to an agent that enhances the permeability so that botulinum toxin can be absorbed, for example when administered to a gastrointestinal tract, to achieve the therapeutic effect. In reference to the disclosure herein, enhancing agent can include dimethylsulfoxide (DMSO), hyaluronidase or a combination of pluronic lecithin organizer (PLO) and DMSO. An enhancing agent may include, and are not limited to, liposomes; transfersomes; lecithin vesicles; ethosomes; water; surfactants, such as anionic, cationic, and nonionic surfactants; polyols; and essential oils.

Local administration: as used herein "local administration" or "locally administering" means direct administration of a pharmaceutical at, or to the vicinity of, a site on or within an animal body, at which site a biological effect of the pharmaceutical is desired. One example of local administration can include direct injection of a botulinum toxin. Topical administration as utilized herein is a type of local administration in which a pharmaceutical agent is applied to a person's gut, such as for example to the luminal wall of a portion of the gastrointestinal tract to which botulinum toxin, for example, is to be administered in accordance with the teachings presented herein.

Neurotoxin: as used herein "neurotoxin" means a biologically active molecule with a specific affinity for a neuronal cell surface receptor. Neurotoxin includes Clostridial toxins, such as Clostridial botulinum toxins, both as pure toxin (having a molecular weight of about 150 kDa) and as complexed with one or more non-toxin, toxin associated proteins; the complexes having molecular weights of about 900 kD, 700, kD, 500 kD or 300 kD, for example. Botulinum toxins can include toxins that are recombinantly made and modified in accordance with known molecular techniques, that is, a modified neurotoxin means a neurotoxin which has had one or more of its amino acids deleted, modified or replaced (as compared to the native neurotoxin) and includes neurotoxins made by recombinant technology as well as derivatives and fragments of a native or recombinantly produced neurotoxin.

Therapeutically effective: as used herein "therapeutically effective" means an amount of toxin administered that will reduce or ameliorate a condition or symptom (in frequency and/or intensity) in a subject. The therapeutically effective amount of botulinum neurotoxin (type A or type B) delivered to a subject is an amount that achieves a desired effect yet does not result in undesirable systemic side effects associated with systemic neurotoxin poisoning, as known by those of ordinary skill in the art.

The neurotoxin (botulinum toxin type A or type B) inhibits gut-derived serotonin production and increases bone density in the mammal, thereby treating osteoporosis in the mammal. In one example, the neurotoxin is administered to the mucosa of gastrointestinal tract or a portion thereof. In a particular embodiment, the neurotoxin is administered to the mucosa of small intestine and in another embodiment, the neurotoxin is administered to the mucosa of the duodenum.

Methods for administering neurotoxin in order to practice particular embodiments disclosed herein, include, but are not limited to, instillation, oral administration, topical administration (that is, to the luminal lining of the gastrointestinal tract or portions thereof), direct injection, implantation of a neurotoxin-containing implant, enema, suppository or any combination thereof. In the case of administration via direct injection, such injections can be performed percutaneously, endoscopically, or a combination thereof. In a most preferred embodiment, the neurotoxin utilized in accordance with the teaching presented herein is a botulinum toxin type A. In particular embodiments the quantity of neurotoxin administered is from about 1 unit to about 50,000 units. As an example, where the neurotoxin is a botulinum toxin type A, the amount administered is from about 1 unit to about 2750 units of the botulinum toxin. In one regimen, neurotoxin is administered about every 1 to about 12 months to the patient in need thereof. The mammal treated can be any mammal, however, is preferably a human.

### DESCRIPTION

The present invention is based on the discovery that gut derived serotonin (GDS) has a profound effect on bone formation, and particularly, that elevated levels of GDS lead to decreased bone density and increased fragility.

GDS is produced by converting L-tryptophan to 5-hydroxy-L-tryptophan (5-HTP) via tryptophan hydroxylase (Tph) and further converting 5-HTP to serotonin (5-HT) via amino acid decarboxylase (DDC). The Tph-mediated reaction is the rate-limiting step in the reaction. There are two isoforms of Tph; Tph1 is found in the gut and other tissues and Tph2 is brain-specific.

Without wishing to be bound by any particular theory, it is thought that the neurotoxin (botulinum toxin type A or type B) inhibits Tph1 in the serotonin production pathway. This inhibition leads to a decrease in the production of GDS and therefore, decreased inhibition of osteoblast activity.

The mammal in need of treatment can be humans, sheep, horses, cattle, pigs, dogs, cats, rats and mice. In one embodiment, the mammal is a human. In certain embodiments, the human is a man or a woman, of any age, race, or ethnicity. In other embodiments, the human is a pre-, peri-, or post-menopausal woman. In yet another embodiment, the human is a man or woman with osteoporosis or at risk for developing osteoporosis. When the man or woman is at risk of developing osteoporosis, the composition can be administered prophylactically to slow the onset or prevent the onset of osteoporosis. In another embodiment, the composition can be administered to a man or woman at risk of developing osteoporosis due to hormonal disorders, medication, or genetic conditions that are associated with the development of osteoporosis. As one example, the human patient can be, or scheduled to take, selective serotonin reuptake inhibitors.

Exemplary, commercially available, botulinum toxin containing compositions include, but are not limited to, BOTOX® (Botulinum toxin type A neurotoxin complex with human serum albumin and sodium chloride) available from Allergan, Inc., of Irvine, California in 100 unit vials as a lyophilized powder to be reconstituted with 0.9% sodium chloride before use), DYSPORT® (Clostridium botulinum type A toxin haemagglutinin complex with human serum albumin and lactose in the formulation), available from Ipsen Limited, Berkshire, U.K. as a powder to be reconstituted with 0.9% sodium chloride before use) and MYOBLOC® (an injectable solution comprising botulinum toxin type B, human serum albumin, sodium succinate, and sodium chloride at about pH 5.6, available from Solstice Neurosciences, Inc., South San Francisco, California). XEOMIN® (a 150 kDa botulinum toxin type A formulation is also available from Merz Pharmaceuticals, Potsdam, Germany) is another useful neurotoxin which can be used as set forth and in accordance with the teachings herein disclosed.

The amount of toxin administered according to a method within the scope of the present disclosure can vary according to the particular condition being treated, including its severity and other various patient variables including size, weight, age, and responsiveness of the particular patient to the botulinum neurotoxin therapy. To guide the practitioner, typically, no less than about 5 units and no more than about 500 units of a botulinum toxin type A (such as BOTOX®) is administered per injection site (e.g. to the small intestine), per patient treatment session. For topical applications, more toxin can be used, as application to the mucosal lining of the gastrointestinal tract, or selected portions thereof, as herein described herein, as absorption is relied upon for uptake rather than direct injection. For a botulinum toxin type A such as DYSPORT®, preferably no less than about 10 units and no more about 2000 units of the botulinum toxin type A are administered per administration or injection site, per patient treatment session. For a botulinum toxin type B such as MYOBLOC®, preferably no less than about 200 units and not more than 50,000 units and preferably not more about 25000 units of the botulinum toxin type B are administered per administration or injection site, per patient treatment session. Less than about 5, 10 or 200 units (of BOTOX®, DYSPORT® and MYOBLOC® respectively) may fail to achieve a desired therapeutic effect, while more than about 500, 2000 or 25,000 units (of BOTOX®, DYSPORT® and MYOBLOC® respectively) may result in clinically observable side effects which can vary depending on administration method, site and particular patient.

For example and in particular embodiments, an implant that slowly releases a therapeutically effective amount of botulinum toxin can obviously contain an amount of toxin (i.e. of units) that may be higher than an amount that is typically administered, directly (e.g., by subdermal injection). As an illustrative example, while 2000 units of BOTOX® may not be desired to be administered at one time to a target via a syringe, yet these same 2000 units, when incorporated into a slow-release implant that is placed adjacent a gastrointestinal portion of the patient in need of botulinum toxin administration in accordance with the present disclosure, can provide slow, long term dosing/release of botulinum neurotoxin in therapeutically effective amounts. An example of a range of units of botulinum toxins delivered orally, via injection, and via implant, according to one embodiment of the invention, is shown in Table 1.

**TABLE 1**

| | ORAL | INJECTION | IMPLANT |
|---|---|---|---|
| BOTOX® | 100-500 | 50-250 | 25-500 |
| DYSPORT® | 300-1500 | 150-750 | 75-1000 |
| XEOMIN® | 100-500 | 50-250 | 25-500 |
| MYOBLOC® | 10,000-50,000 | 5,000-25,000 | 2500-25,000 |

Formulation of botulinum toxin for ingestion is known in the art (see for example, published U.S. Patent Application No. 20040086532, published May 6, 2004 and U.S Patent 7,238,357) and formulation of orally ingested pharmaceuticals that release actives in such a formulation at particular gastrointestinal regions are known in the art, such as those known medications for the treatment of indigestion, heartburn and "enteric" coated meds as well as extended release (XR), controlled release (CR) forms of medications, such those, for example, that comprise microspheres that release their medicaments in the duodenum. Examples can include, but are not limited to, formulations for tablets containing, in addition to botulinum toxin, starch, hydrogel, lactose, magnesium stearate. Pharmaceutical compositions formulated to administer medications to the duodenum are known in the art, such as shown in U.S. Patent 6,582,720.

In additional embodiments, no less than about 10 units and no more about 400 units of BOTOX®; no less than about 30 units and no more than about 1600 units of DYSPORT®, and; no less than about 250 units and no more than about 20,000 units of MYOBLOC® are administered per site, per patient treatment session.

In still further embodiments, no less than about 20 units and no more about 300 units of BOTOX®; no less than about 60 units and no more than about 1200 units of DYSPORT®, and; no less than about 1000 units and no more than about 15000 units of MYOBLOC® are administered per site, per patient treatment session. There can be multiple injection sites (i.e. a pattern of injections) for each patient treatment session in order to distribute the neurotoxin over a desired target area, such as around/throughout the gut, particularly the small intestine, more particularly, the duodenum.

Although examples of routes of administration and dosages are provided, the appropriate route of administration and dosage are generally determined on a case-by-case basis by the attending physician, as known in the botulinum toxin arts, and titration of the dosage to a therapeutically effective one, for a particular patient/condition, is routinely undertaken. Such determinations are routine to one of ordinary skill in the art (see for example, Harrison's Principles of Internal Medicine (1998), edited by Anthony Fauci et al., 14th edition, published by McGraw Hill and product inserts for BOTOX®, MYOBLOC® and DYSPORT®, where exemplary dose-finding methods are described). For example, the route and dosage for administration of a Clostridial neurotoxin according to the present disclosed invention can be selected based upon criteria such as the solubility characteristics of the neurotoxin chosen as well as the intensity and scope of the condition to be treated.

Additionally, in some embodiments, a physician may have to alter dosage in each case (i.e. patient) in accordance with the assessment of the severity of the condition, as typically done when treating patients with a condition/disorder. Further, in some embodiments, the treatment may have to be repeated at least one additional time, in some cases several times, depending on the severity of the condition and the patient's overall health. If, for example, a patient is not deemed physically suitable for a full administration of botulinum toxin, or if a full administration is not desired for any reason, smaller doses on multiple occasions may prove to be efficacious. Further still, if botulinum toxin is administered at a certain dosage that is not sufficient to attain the desired treatment goal, such as reduction in an amount GDS produced over a particular time period, the dose may be increased for a second and subsequent administration session(s) by the attending physical as he/she best sees fit (i.e. a dose finding regimen).

In practicing the methods disclosed herein, the compounds may be used alone or in combination, or in combination with other therapeutic or diagnostic agents. The composition of this invention may be co-administered along with other compounds/compositions typically prescribed for the condition(s), such as osteoporosis, according to generally accepted medical practice.

Although one exemplary composition may only contain a single type of neurotoxin, such as botulinum toxin type A, as the active ingredient to modulate bone formation, other therapeutic compositions may include two or more types of neurotoxins, which may provide enhanced therapeutic effects of the disorders. For example, a composition administered to a patient may include botulinum toxin type A and botulinum toxin type B. Administering a single composition containing two different neurotoxins may permit the effective concentration of each of the neurotoxins to be lower than if a single neurotoxin is administered to the patient while still achieving the desired therapeutic effects.

In certain embodiments, it is desirable for the neurotoxin to be administered to the gastrointestinal tract. In another embodiment, the neurotoxin is administered to the small intestine and in yet another embodiment, the neurotoxin is administered to the duodenum. In one embodiment, the neurotoxin is administered to the luminal surface of the duodenum. In yet another embodiment, the neurotoxin is administered to the mucosa of the duodenum. In this embodiment, care is taken to avoid the major duodenal papilla, found toward the distal portion of the duodenum and the anatomic structure from which bile and pancreatic secretions are released. In one embodiment, about 50 to about 200 units of neurotoxin are distributed, via injection for example, at about 10 to about 20 sites along the luminal surface of the duodenum.

The neurotoxin can be administered to the gastrointestinal tract using one or more routes, including but not limited to, orally, topically, by implant, by instillation via intestinal tube or catheter passed intranasally or intratracheally, endoscopically, or percutaneously (by injection). Visual guidance may be required and can be provided by radiograph, fluoroscopy, CT, MRI, ultrasound, and a combination thereof. These techniques of administration and guidance are well known to those of skill in the art.

In certain embodiments the neurotoxin is administered orally. The therapeutic dose of orally administered neurotoxin is such that there are nominal or insignificant systemic effects due to any neurotoxin that is absorbed through the gut lining in to the circulatory system (for details, see U.S. Published Patent Application No. 2007/0269463). An orally administered neurotoxin can remain bioactive in the harsh environment of the gastrointestinal tract, and is prepared so that the neurotoxin is substantially uniformly dispersed in a biodegradable carrier. An alternate oral formulation can comprise a carrier coated by a biodegradable coating, and either the thickness of the coating or the coating material can be varied. Further, the neurotoxin can be in a controlled-release composition. The thickness of the oral formulation can be used to control the absorption of water by, and thus the rate of release, of a neurotoxin from the formulation. Thicker oral formulations release botulinum toxin more slowly than thinner ones. The oral formulations include excipients, such as bulking agents, stabilizing agents, buffers, etc. The oral formulation can be in any suitable oral form, including but not limited to a capsule, tablet, microspheres, gelcap, and a liquid.

In embodiments where the neurotoxin is administered by implant, release of the neurotoxin is achieved over a period of time, from hours, to days, to weeks. For example, the neurotoxin can be released from about 1 hour to about 4, or 8, or 12 weeks. Monophasic or pulsatile release of the neurotoxin can be achieved utilizing one or more implants. In certain embodiments, a plurality of implants can be used with the same or differing carrier material compositions. Further, the neurotoxin can be encapsulated in one or more types of microspheres having the same or differing degradation patterns such that the neurotoxin can be released at similar or differing rates, respectively. In one embodiment, one or more implants are implanted within the luminal wall of the duodenum. Additional detail regarding botulinum implants is provided in U.S. Patents Nos. 6,306,423, 6,506,399, and published U.S. Patent Application 20070020295, filed January 25, 2007.

The botulinum toxins described herein can be incorporated into a topical formulation, as known in the art. Preferably, the compositions can more easily allow the application of the botulinum toxin into the target site in a patient. Suitable compositions include, but are not limited to creams, lotions, hydrogels, jellies, sprays, pastes, adhesives, emulsions, nanoparticles, microparticles, drops, powders, and combinations thereof. Such botulinum toxin preparations can be delivered to the desired gastrointestinal target location, such as the duodenum, via an endoscope, for example. In particular examples, a botulinum toxin can be delivered via injection to attenuate gut-derived serotonin production. Methods for administering a botulinum toxin to the gastrointestinal tract are well known in the art, (e.g. as disclosed in U.S. Patent 5,437,291 and "Botulinum toxin for spastic GI disorders: A systematic review"; Gastrointestinal Endoscopy Volume 57, Issue 2, February 2003, pages 219-235, both herein incorporated by reference in their entirety), and such apparatus can be utilized in order to practice particular embodiments in accordance with the teachings provided herein.

The botulinum toxins used herein inhibit gut-derived serotonin production. The suppressive effects provided by the toxin can persists for several months, such as from about 1 month to about 12 months, or from about 1 month to about 6 months. In one embodiment, the suppression can last for years, for example up to about 2 years, for example, if the botulinum toxin is supplied in a slow release implant form, for example.

### EXAMPLE 1

### Treatment of osteoporosis with botulinum toxin A

A 56 year old woman presents with decreased bone mineral density (BMD) indicative of osteoporosis. The patient is administered 150 units of botulinum toxin A, endoscopically, at 15 sites distributed along the luminal surface of the duodenum. After a period of 3 months, the patient shows increased BMD, indicative of new bone deposition.

### EXAMPLE 2

### Treatment of osteoporosis with botulinum toxin B

A 70 year old man presents with an idiopathic decrease in BMD. Patient is orally administered 25,000 units of botulinum toxin B every 6 months for a period of 1.5 years. Patient is evaluated 2 years from the date of the first treatment and shows increased BMD indicative of new bone deposition.

### EXAMPLE 3

### Treatment of osteoporosis in a patient utilizing selective serotonin reuptake inhibitor (SSRI)

A post-menopausal 55 year old woman presents at her physician's office reporting that for reasons unknown to her, she often has feelings of agitation, irritation and depression and on some days, comes to experience states of anxiety lasting anywhere from a few hours to a few days. After a question and answer session and physical workup, the physician decides to prescribe the selective serotonin reuptake inhibitor, fluoxetine (Prozac®), at a dosage of 10mg per day. Considering the patient's age and as part of the workup, a bone mineral density (BMD) test is undertaken, providing a normal T-score of 0.2. The patient takes the fluoxetine and reports she no longer has feelings of agitation, irritation and depression but however experiences pain in her hips and legs. The doctor orders another BMD test, where the results show a T-score of -3.3, which indicates osteoporosis. The physician proceeds to endoscopically administer to the duodenum (taking care to avoid the major duodenal papilla) about 100 units of a botulinum toxin type A (BOTOX®) at 10 sites. The patient undergoes another (BMD) test about 4 months later, receiving a T-score of -0.5, evidencing normal bone density.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however; inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context. Each and every feature described herein, and each and every combination of two or more of such features, is included within the scope of the present invention provided that the features included in such a combination are not mutually inconsistent. In addition, any feature or combination of features may be specifically excluded from any embodiment of the present invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

Furthermore, numerous references have been made to patents and printed publications throughout this specification. Each of the above-cited references and printed publications are individually incorporated herein by reference in their entirety.

In closing, it is to be understood that the embodiments of the invention disclosed herein are illustrative of the principles of the present invention.

## Claims

1. A composition comprising a neurotoxin for use in a method of treating osteoporosis in a mammal, wherein the neurotoxin is selected from botulinum toxin types A and B.

2. A composition for use according to claim 1, wherein the neurotoxin is to be administered to the mucosa of the gastrointestinal tract.

3. A composition for use according to claim 2, wherein the neurotoxin is to be administered to the mucosa of the small intestine.

4. A composition for use according to claim 3, wherein the neurotoxin is to be administered to the mucosa of the duodenum.

5. A composition for use according to claim 2, wherein the neurotoxin is to be administered via instillation, orally, topically, via injection, via implant, or a combination thereof.

6. A composition for use according to claim 5, wherein the injection is to be administered percutaneously, endoscopically, or a combination thereof.

7. A composition for use according to claim 4, wherein the neurotoxin is botulinum toxin type A.

8. A composition for use according to claim 1, wherein the neurotoxin is botulinum toxin type A and is to be administered in an amount of from about 1 unit to about 2750 units.

9. A composition for use according to claim 8, wherein the neurotoxin is to be administered about every 1 to 12 months.

10. A composition for use according to claim 1, wherein the quantity of neurotoxin to be administered is about 1 unit to about 50,000 units.

11. A composition for use according to claim 1, wherein the mammal is a human.

12. A composition for use according to claim 1 or claim 7, wherein the neurotoxin is to be administered to the gastrointestinal tract.

## Patentansprüche

1. Zusammensetzung umfassend ein Neurotoxin zur Verwendung in einem Verfahren zur Behandlung von Osteoporose bei einem Säuger, worin das Neurotoxin ausgewählt ist aus den Botulinumtoxin-Typen A und B.

2. Zusammensetzung zur Verwendung gemäß Anspruch 1, worin das Neurotoxin über die Schleimhaut des Verdauungstrakts verabreicht werden soll.

3. Zusammensetzung zur Verwendung gemäß Anspruch 2, worin das Neurotoxin über die Schleimhaut des Dünndarms verabreicht werden soll.

4. Zusammensetzung zur Verwendung gemäß Anspruch 3, worin das Neurotoxin über die Schleimhaut des Zwölffingerdarms verabreicht werden soll.

5. Zusammensetzung zur Verwendung gemäß Anspruch 2, worin das Neurotoxin durch Einträufeln, oral, topisch, durch Injektion, durch ein Implantat oder eine Kombination davon verabreicht werden soll.

6. Zusammensetzung zur Verwendung gemäß Anspruch 5, worin die Injektion perkutan, endoskopisch oder durch eine Kombination davon verabreicht werden soll.

7. Zusammensetzung zur Verwendung gemäß Anspruch 4, worin das Neurotoxin Botulinumtoxin des Typs A ist.

8. Zusammensetzung zur Verwendung gemäß Anspruch 1, worin das Neurotoxin Botulinumtoxin vom Typ A ist und in einer Menge von etwa 1 Einheit bis etwa 2750 Einheiten verabreicht werden soll.

9. Zusammensetzung zur Verwendung gemäß Anspruch 8, worin das Neurotoxin etwa alle 1 bis 12 Monate verabreicht werden soll.

10. Zusammensetzung zur Verwendung gemäß Anspruch 1, worin die Menge des zu verabreichenden Neurotoxins etwa 1 Einheit bis etwa 50.000 Einheiten beträgt.

11. Zusammensetzung zur Verwendung gemäß Anspruch 1, worin der Säuger ein Mensch ist.

12. Zusammensetzung zur Verwendung gemäß Anspruch 1 oder Anspruch 7, worin das Neurotoxin in den Verdauungstrakt verabreicht werden soll.

## Revendications

1. Composition comprenant une neurotoxine, pour son utilisation dans un procédé de traitement de l'ostéoporose chez un mammifère, dans laquelle la neurotoxine est choisie parmi les toxines botuliques de types A et B.

2. Composition, pour son utilisation selon la revendication 1, dans laquelle la neurotoxine doit être administrée sur la muqueuse des voies gastro-intestinales.

3. Composition pour son utilisation selon la revendication 2, dans laquelle la neurotoxine doit être administrée sur la muqueuse de l'intestin grêle.

4. Composition pour son utilisation selon la revendication 3, dans laquelle la neurotoxine doit être administrée sur la muqueuse du duodénum.

5. Composition pour son utilisation selon la revendication 2, dans laquelle la neurotoxine doit être administrée par instillation, par voie orale, topique, par injection, par un implant ou une combinaison de ceux-ci.

6. Composition pour son utilisation selon la revendication 5, dans laquelle l'injection doit être administrée par voie percutanée, endoscopique ou une combinaison de celles-ci.

7. Composition pour son utilisation selon la revendication 4, dans laquelle la neurotoxine est une toxine botulique de type A.

8. Composition pour son utilisation selon la revendication 1, dans laquelle la neurotoxine est une toxine botulique de type A et doit être administrée en une quantité d'environ 1 unité à environ 2750 unités.

9. Composition pour son utilisation selon la revendication 8, dans laquelle la neurotoxine doit être administrée à peu près tous les 1 à 12 mois.

10. Composition pour son utilisation selon la revendication 1, dans laquelle la quantité de neurotoxine qui doit être administrée est d'environ 1 unité à environ 50 000 unités.

11. Composition pour son utilisation selon la revendication 1, le mammifère étant un être humain.

12. Composition pour son utilisation selon la revendication 1 ou la revendication 7, dans laquelle la neurotoxine doit être administrée dans les voies gastro-intestinales.
